(19) Europäisches Patentamt
European Patent Office
Office européen des brevets

(11) Numéro de publication: **0 022 038**
**B1**

# (12) FASCICULE DE BREVET EUROPEEN

(45) Date de publication du fascicule du brevet: **15.09.82**

(51) Int. Cl.³: **C 07 C 29/32**, C 07 C 31/08

(21) Numéro de dépôt: **80420066.5**

(22) Date de dépôt: **04.06.80**

## (54) Procédé d'homologation du méthanol

(30) Priorité: **07.06.79 FR 7915079**

(43) Date de publication de la demande:
**07.01.81 Bulletin 81/1**

(45) Mention de la délivrance du brevet:
**15.09.82 Bulletin 82/37**

(84) Etats contractants désignés:
**AT BE CH DE FR GB IT LI LU NL SE**

(56) Documents cités:
EP - A - 0 013 464
DE - C - 877 598
FR - A - 1 341 840
GB - A - 2 007 652
US - A - 3 285 948
US - A - 4 133 966
BULLETIN OF THE CHEMICAL SOCIETY OF JAPAN, vol. 52, no. 2, février 1979, Tokyo, JP T. MIZOROKI et al. "Further study of methanol carbonylation catalyzed by cobalt, rhodium and iridium catalysts", pages 479—482.
CHEMICAL ABSTRACTS, vol. 87, no. 19, 7 novembre 1977, page 545, abrégé no 151697e Columbus, Ohio, USA.

(73) Titulaire: **RHONE-POULENC CHIMIE DE BASE**
**25, quai Paul Doumer**
**F-92408 Courbevoie (FR)**

(72) Inventeur: **Gauthier-Lafaye, Jean**
**21, rue Duguesclin**
**F-69006 Lyon (FR)**
Inventeur: **Perron, Robert**
**La Pecolière**
**F-69390 Charly (FR)**

(74) Mandataire: **Varniere-Grange, Monique et al,**
**RHONE-POULENC RECHERCHES Centre de Recherches de Saint-Fons Service Brevets B.P. 62**
**F-69190 Saint-Fons (FR)**

**EP 0 022 038 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé d'homologation du méthanol

La présente invention a pour objet un procédé perfectionné de préparation de l'éthanol par hydrocarbonylation du méthanol, en présence d'un catalyseur à base de cobalt.

L'éthanol, produit industriel de grand intérêt a été préparé de manière classique par fermentation de diverses substances naturelles, ou par hydratation, directe ou indirecte, de l'éthylène. De nombreux procédés de synthèse industrielle ont été développés qui mettent en oeuvre des charges d'éthylène, dérivé du pétrole. Toutefois, depuis un certain nombre d'années on perçoit un besoin croissant de procédés de synthèse des grands produits chimiques, au départ de matières premières ne provenant pas du pétrole. C'est pourquoi on peut constater un intérêt de plus en plus vif pour la synthèse de produits chimiques à partir du gaz de synthèse, mélange de monoxyde de carbone et d'hydrogène.

Dans ce contexte le méthanol, qui peut être produit par réaction de monoxyde de carbone et de l'hydrogène, et qui à son tour, peut être mis à réagir sur le gaz de synthèse en présence de cobalt, pour former de l'éthanol, revêt une importance toute particulière.

La réaction de méthanol sur le gaz de synthèse en vue de la formation de l'éthanol, réaction encore appelée homologation du méthanol, a constitué et continue d'être l'objet de nombreuses recherches.

Ainsi, I. WENDER et ses collaborateurs ("Science", volume 113 page 206, 1951) ont montré que le méthanol réagit avec un mélange équimolaire de monoxyde de carbone et d'hydrogène à 185°C, sous 360 atmosphères en présence de dicobaltoctacarbonyle. Dans ces conditions, on obtient un mélange de divers produits, renfermant de l'éthanol avec une sélectivité médiocre; l'intérêt de cette technique est resté purement académique, la productivité horaire d'un tel catalyseur étant dérisoire.

D'autres auteurs (Cf. brevet français 1.323.453) ont pu améliorer sensiblement la productivité en éthanol, en conduisant cette réaction à 400 bars, et vers 200°C en présence simultanée d'acétate de cobalt et d'iode, le rapport molaire $CO/H_2$ étant égal à 0,5.

Les mêmes auteurs ont par la suite élevé encore nettement cette productivité en conduisant la réaction d'homologation dans les conditions rappelées ci-avant, en ajoutant une infime proportion d'un halogénure de ruthénium au système catalytique à base d'acétate de cobalt et d'iode. (Cf. Brevet des ETATS UNIS D'AMERIQUE no. 3.285.948). En effet, les meilleurs résultats sont obtenus avec environ 0,05 à 0,12 atome-gramme de ruthénium pour un atome-gramme de cobalt et correspondent à des productivités de l'ordre de 350 à 400 g d'éthanol par heure et par litre de milieu réactionnel et de l'ordre de 200 g d'éthanol par heure et par gramme de cobalt. (Cf. Les exemples n°. 4, 7 et 12 du brevet américain précité).

Néanmoins, la possibilité d'exploitation industrielle d'un tel procédé est compromise par la pression élevée nécessaire pour atteindre des productivités horaires acceptables.

Il a maintenant été trouvé de maniére tout á fait inattendue qu'il est possible d'homologuer le méthanol, sous une pression totale inférieure à 400 bars, avec une productivité horaire acceptable en éthanol, en présence d'un catalyseur à base de cobalt.

La présente invention a donc pour objet un procédé d'hydrocarbonylation du méthanol en présence simultanée d'une quantité efficace de cobalt, d'au moins un halogénure ionique, d'au moins un halogénure d'alkyle, et d'au moins 2 atomes-grammes de ruthénium par atome-gramme de cobalt.

Les recherches, qui ont abouti à la présente invention, ont montré d'une mainère en soi remarquable que l'adjonction au système catalytique à base de cobalt et de ruthénium, d'un halogénure ionique et d'un halogénure d'alkyle, le rapport Ru/Co étant supérieur à deux, accroît substantiellement l'activité d'un tel système, ce qui permet de réaliser l'homologation du méthanol sous une pression totale de l'ordre de 250 bars environ, tout en ayant une productivité horaire satisfaisante.

Le procédé selon l'invention exige la mise en oeuvre d'au moins un halogénure ionique. Par halogénure ionique on entend les chlorures, bromures, ou, de préférence, les iodures minéraux ou organiques dont les cations sont choisis parmi les cations des métaux alcalins, les cations des métaux alcalino-terreux et les cations ammonium ou phosphonium quaternaire représentés par les formules I à III ci-après:

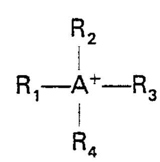

(I)

dans laquelle A représente un atome d'azote ou de phosphore, $R_1$, $R_2$, $R_3$ et $R_4$, pouvant être identiques ou différents représentent l'hydrogène ou, de préférence, des radicaux organiques dont la valence libre est portée un atome de carbone, deux quelconques de ces divers radicaux pouvant éventuellement former ensemble un radical unique divalent.

Plus spécifiquement $R_1$, $R_2$, $R_3$ et $R_4$ peuvent représenter des radicaux alkyles linéaires ou ramifiés, des radicaux cycloalkyles, aralkyles (par exemple benzyle) ou aryles monocycliques, ayant au plus 16 atomes de carbone, pouvant le cas échéant être substitués par 1 à 3 radicaux

alkyles ayant de 1 à 4 atomes de carbone; deux des radicaux $R_1$ à $R_4$ pouvant éventuellement former ensemble un radical unique divalent — alkylène ou alcénylène — comportant 3 à 6 atomes de carbone (par exemple un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

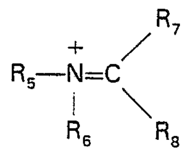 (II)

dans laquelle $R_5$, $R_6$, $R_7$ et $R_8$ identiques ou différents représentent des radicaux alkyles ayant de 1 à 4 atomes de carbone, l'un des radicaux $R_7$ ou $R_8$ pouvant en outre représenter l'hydrogène, $R_7$ et $R_8$ pouvant éventuellement former ensemble un radical unique divalent alkylène comportant de 3 à 6 atomes de carbone, tétraméthylène ou hexaméthylène par exemple; $R_6$ et $R_7$ ou $R_8$ peuvent former ensemble un radical unique divalent—alkylène ou alcénylène—comportant 4 atomes de carbone et le cas échéant 1 ou 2 doubles liaisons éthylèniques, l'atome d'azote étant alors inclus dans un hétérocycle, pour constituer, par exemple, un cation pyridinium.

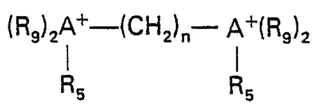 (III)

dans laquelle $R_5$ et. $A^+$ ont la signification donnée précédemment, $R_9$ pouvant être identique à $R_5$ représente un radical alkyle ayant de 1 à 4 atomes de carbone ou un radical phényle, et n est un entier compris entre 1 et 10 ($1 \leqslant n \leqslant 10$) et de préférence entre 1 et 6 ($1 \leqslant n \leqslant 6$). A titre d'exemple d'halogénures d'ammonium quatemaire convenant à la mise en oeuvre du présent procédé on peut citer les chlorures, les bromures et, plus particulièrement les iodures

de tétraméthylammonium,
de triéthylméthylammonium,
de tributylméthylammonium,
de triméthyl(n-propyl)ammonium,
de tétraéthylammonium,
de tétrabutylammonium,
de dodécyltriméthylammonium,
de benzyltriméthylammonium,
de benzyldiméthylpropylammonium,
de benzyldiméthyloctylammonium,
de diméthyldiphénylammonium,
de méthyltriphénylammonium,
de N,N-diméthyl-triméthylénammonium,
de N,N-diéthyl-triméthylénammonium,
de N,N-diméthyl-tétraméthylénammonium,
de N,N-diéthyl-tétraméthylénammonium,
de N-méthylpyridinium
de N-éthylpyridinium
de N-méthylpicolinium

A titre d'exemple d'halogénures de phosphonium quaternaire convenant également à la mise en oeuvre du présent procédé, on peut citer les chlorures, les bromures et, plus particulièrement les iodures

de tétraméthylphosphonium,
d' éthyltriméthylphosphonium
de triméthylpentylphosphonium,
d' octyltriméthylphosphonium,
de dodécyltriméthylphosphonium,
de triméthylphénylphosphonium,
de diéthyldiméthylphosphonium,
de dicyclohexyldiméthylphosphonium,
de diméthyldiphénylphosphonium,
de cyclohexyltriméthylphosphonium,
de triéthylméthylphosphonium,
de méthyl-tri(isopropyl)phosphonium,
de méthyl-tri(n-propyl)phosphonium,
de méthyl-tri (n-butyl)phosphonium,
de méthyl-tris(méthyl-2 propyl)phosphonium,
de méthyltricyclohexylphosphonium,
de méthyltriphénylphosphonium,
de méthyltribenzylphosphonium,
de méthyl-tris (méthyl-4 phényl)phosphonium,
de méthyltrixylylphosphonium,
de diétylméthylphénylphosphonium,
de dibenzylméthylphénylphosphonium,
d' éthyltriphénylphosphonium,
de tétraéthylphosphonium,
d' éthyl-tri(n-propyl)phosphonium,
de triétylpentylphosphonium,
d' éthyltriphénylphosphonium,
de n-butyl-tri-(n-propyl)phosphonium,
de butyltriphénylphosphonium,
de benzyltriphénylphosphonium,
de ($\beta$-phénylléthyl)diméthylphénylphosphonium,
de tétraphénylphosphonium,
de triphényl(méthyl-4 phényl)phosphonium.

La nature précise du cation ammonium ou phosphonium quaternaire n'est pas fondamentale dans le cadre du présent procédé. Le choix parmi ces composés est davantage orienté par des considérations d'ordre pratique, telles que la solubilité dans le milieu réactionnel, la disponibilité et la commodité d'emploi.

A ce titre, les halogénures d'ammonium ou de phosphonium quaternaire représentés soit par la formule (I) dans laquelle l'un quelconque des radicaux $R_1$ à $R_4$ est choisi parmi les radicaux alkyles linéaires ayant de 1 à 4 atomes de carbone, soit par les formules (II) ou (III) dans laquelle $R_5$ (ou$R_6$) est également un radical alkyle ayant de 1 à 4 atomes de carbone, conviennent particulièrement bien.

En outre, parmi les halogénures d'ammonium on préfère ceux dont les cations correspondent à la formule I dans laquelle tous les radicaux $R_1$

à $R_4$ sont choisis parmi les radicaux alkyles linéaires ayant de 1 à 4 atomes de carbone et dont au moins trois d'entre eux sont identiques.

De même, parmi les halogénures de phosphonium quaternaire, on préfère ceux dont les cations correspondent à la formule (I) dans laquelle l'un quelconque des radicaux $R_1$ à $R_4$ représente un radical alkyle linéaire ayant de 1 à 4 atomes de carbone, les trois autres radicaux étant identiques et choisis parmi les radicaux phényle, tolyle ou xylyle.

Les iodures de phosphonium quaternaire et plus particulièrement ceux dont les cations correspondent à la formule (I) ci-avant, dans laquelle l'une des radicaux $R_1$ à $R_4$ est un radical alkyle ayant de 1 à 4 atomes de carbone, les trois autres radicaux étant identiques et choisis parmi les radicaux phényl, tolyle ou xylyle, constituent une class d'halogénures ioniques particulièrement commodes pour la mise en oeuvre de la présente invention.

Un mode préféré de réalisation de la présente invention comprend l'utilisation d'iodures des métaux alcalins ou alcalino-terreux, tels que: LiI, NaI, KI, CsI, $CaI_2$ et $MgI_2$. De préférence on utilise un ou plusieurs iodures des métaux alcalins et de manière encore plus avantageuse, NaI ou KI.

Selon la présente invention, le rapport molaire $X^-/Co$, $X^-$ provenant de l'halogénure ionqiue doit être au moins égal à 5. Il n'est pas souhaitable que ce rapport dépasse la valeur de 200. Des résultats trés satisfaisants sont obtenus pour un rapport $X^-/Co$ de l'ordre de 10 à 100.

Le procédé selon l'invention exige également la mise en oeuvre d'au moins un halogénure d'alkyle, c'est-à-dire d'un composé de formule RX dans laquelle X représente un atome de chlore, de brome ou, de préférence un atome d'iode et R est un radical alkyle ayant au maximum 16 atomes de carbone. Bien entendu les halogénures de méthyle qui peuvent être engagés initialement dans le milieu réactionnel, sont susceptibles d'être formés in situ à partir de dérivés halogénés tels que $Cl_2$, $Br_2$, $I_2$, HCl, HBr, HI, $CoBr_2$, $CoI_2$, $RuCl_3$ et $RuI_3$ et du méthanol (matière de départ). En d'autres termes une partie ou la totalité de l'halogénure de méthyle utile à la mise en oeuvre du présent procédé peut être formée à partir des précurseurs définis ci-avant.

On constatera en outre que lorsque le dérivé halogéné est choisi parmi les composés du cobalt ou du ruthénium, il peut être considéré comme précurseur non seulement de l'halogénure de méthyle, mais encore comme précurseur du (ou des) catalyseur(s) métallique(s). Dans ce cas particulier il s'avère préférable de charger en outre, initialement, un halogénure d'alkyle et/ou un précurseur de l'halogénure de méthyle, distinct des halogénures métalliques en cause.

L'invention envisage notamment l'utilisation des chlorures, bromures et iodures d'alkyles inférieurs ayant de 1 à 4 atomes de carbone dans la molécule, tels que le bromure et l'iodure de méthyle, le bromure et l'iodure d'ethyle.

De préférence, on utilise l'iodure de méthyle et/ou l'une de ses sources potentielles choisies parmi l'iode, l'acide iodhydrique, l'iodure de cobalt, et l'iodure de ruthénium.

Selon la présente invention le rapport molaire X/Co, X provenant de l'halogénure d'alkyle est au moins égal à 2. Il n'est pas souhaitable de dépasser la valeur de 100 pour ce rapport, notamment pour des raisons technologiques, en particulier pour limiter les risques de corrosion de l'appareillage. De bons résultats sont obtenus pour un rapport X/Co de l'ordre de 10 à 50.

Le procédé selon l'invention est réalisé en présence de cobalt. N'importe quelle source de cobalt susceptible de réagir avec l'oxyde de carbone dans le milieu de réaction pour donner des complexes carbonyles du cobalt peut être utilisée dans le cadre de la présente invention.

Des sources typiques de cobalt sont par exemple le cobalt métallique finement divisé, des sels inorganiques tel que le carbonate de cobalt, des sels organiques, en particulier d'un acide gras. Peuvent également être employés les cobalt-carbonyles, hydrocarbonyles, ou leurs complexes. Parmi les dérivés du cobalt concenant pour la mise en oeuvre du procédé selon l'invention, on peut citer l'acétate et le formiate de cobalt, les halogénures de cobalt, en particulier l'iodure de cobalt, le dicobalt-octacarbonyle.

On opère avec une quantité efficace de cobalt. En général cette quantité est comprise entre 0,1 et 100, et de préférence, entre 0,5 et 50 milliatomes-grammes de cobalt par litre de milieu réactionnel.

Le procédé selon l'invention nécessite également la présence de ruthénium. La forme précise sous laquelle le ruthénium est engagé à la réaction n'est pas fondamentale, dans le cadre de la présente invention. On peut utiliser le ruthénium métallique sous forme finement divisée, ou des composés du ruthénium, tels que $RuCl_3$, $RuI_3$, $RuO_2$, $Ru_3(CO)_{12}$ et $Ru(C_5H_7O_2)_3$.

La quantité de ruthénium à mettre en oeuvre dans le cadre du présent procédé doit être d'au moins 2 atomes-grammes de ruthénium par atome-gramme de cobalt engagé à la réaction. De préférence, le rapport du ruthénium au cobalt est supérieur ou égal à 5. Il n'est pas utile de dépasser la valeur de 20 pour ce rapport.

Le procédé de carbonylation selon la présente invention est conduit de préférence en phase liquide. Comme on opère le plus souvent avec du méthanol en excès, l'emploi simultané d'un solvent supplémentaire est généralement superflu mais on peut en principe faire usage de tels solvants, par exemple d'hydrocarbures, d'esters, d'éthers et des produits de la réaction.

Dans le cadre du présent procédé, il n'est pas nécessaire de purifier ou de déshydrater le méthanol au préalable. On peut utiliser du

méthanol de qualité technique.

Selon le présent procédé, on fait réagir sur le méthanol un mélange de monoxyde de carbone et d'hydrogène. Il est essentiel que ledit mélange renferme au moins 25% en mole d'hydrogène. En général, on peut utiliser des mélanges renfermant jusqu'à 95% d'hydrogène. On utilise de préférence des mélanges renfermant de 40 à 80% d'hydrogène. Le mélange de gaz peut renfermer des impuretés telles que, par exemple, du dioxyde de carbone, de l'oxygène, du méthane et de l'azote.

La réaction est mise en oeuvre sous une pression totale généralement comprise entre 50 et 400 bars. De préférence cette pression est comprise entre 100 et 350 bars.

La température de réaction est d'au moins 180°C environ et peut atteindre 240°C lorsqu'on opère sans solvant. Lorsqu'on met en oeuvre un solvant, ce qui demeure facultatif dans le cadre de la présente invention la température peut atteindre 300°C. De préférence on opère dans la gamme de température allant de 200 à 240°C.

Les exemples ci-après illustrent la présente invention sans toutefois en limiter le domaine ou l'esprit.

N.B. Par éthanol "réalisable" on entend la quantité totale d'éthanol libre ou bloqué sous forme d'éthers et d'esters.

Exemple 1

Dans un autoclave en acier inoxydable Z8—CNDT 17—12 (norme AFNOR), de 250 ml de capacité, on charge: 95 ml de méthanol, 5 ml d'eau, 521 mg d'iodure de méthyle (3,67 m Mol), 1,8 g d'iodure de sodium (12 m Mol), 0,127 m At-g de cobalt sous la forme de dicobaltoctacarbonyle et 0,657 m At-g de ruthénium sous la forme de triruthénium dodécacarbonyle. Après fermeture de l'autoclave on établit une pression de 140 bars à l'aide d'un mélange $CO/H_2$ équimoléculaire. L'agitation par un système de va et vient est alors mise en route et l'autoclave est porté à 215°C, en 25 minutes environ. La pression dans l'autoclave atteint alors 210 bars et elle est maintenue entre 220 et 260 bars par des recharges périodiques d'un mélange $CO/H_2$ : 1/2. Après 1 heure 15 minutes de réaction à la température indiquée, le chauffage et l'agitation sont arrêtés; l'autoclave est refroidi et dégazé. Le mélange réactionnel est dilué et analysé par chromatographie gazeuse.

Il contient 15,2 g d'ethanol, 4,48 g d'oxyde de méthyle et d'éthyle et 0,88 g d'éther diéthylique.

La productivité exprimée par rapport à l'éthanol réalisable est de 160 g par heure et par litre, et de 2100 g par heure et par gramme de cobalt.

Les essais ci-après ne rentrent pas dans le cadre de la présente invention et ne sont donnés qu'à titre comparatif.

*Essai témoin a:*

On a reproduit l'exemple 1 ci-avant en l'absence de triruthénium dodécacarbonyle: on n'a pas obtenu d'éthanol.

*Essai témoin b:*

On a reproduit l'exemble 1 ci-avant en l'absence de dicobaltoactacarbonyl (la pression étant maintenue entre 220 et 260 bars par des recharges périodiques d'un mélange $CO/H_2$ équimoléculaire). Après 1 heure 30 minutes de réaction on a obtenu 1,63 g d'éthanol et 0,83 d'oxyde de méthyle et d'éthyle.

*Essai témoin c:*

On a reproduit l'exemple 1 ci-avent en ne chargeant que 0,117 m At-g de ruthénium sous forme de triruthénium dodécacarbonyle. On a obtenu 6 g d'éthanol et 2,15 g d'oxyde de méthyle et d'éthyle. La productivité en éthanol réalisable n'est que de 55 g/h x 1.

Exemple 2

En suivant le mode opératoire décrit précédemment on a chargé 0,118 m At-g de cobalt sous forme de dicobalt octacarbonyle, 0,94 m At-g de ruthénium sous forme de triruthénium dodécacarbonyle, 12 m Mol d'iodure de potassium, 4,67 m Mol d'iodure de méthyle et 100 ml de méthanol. La température était de 205°C et la pression dans l'autoclave a été maintenue entre 220 et 250 bars par des recharges périodiques d'un mélange $CO/H_2$ : 2/3. En une heure de réaction on a obtenu 15,1 g d'éthanol et 7,33 g d'oxyde de méthyle et d'éthyle. La productivité en éthanol réalisable est de 210 g par heure par litre et 3000 g par heure par gramme de cobalt.

Exemple 3

En suivant le mode opératoire décrit précédemment on a chargé 3 m Mol d'iodure de sodium, 6,8 m Mol d'iodure de méthyle, 95 ml de méthanol, 5 ml d'eau, 2,64 m At-g de ruthénium sous forme de triruthénium dodécacarbonyle, et 0,48 m At-g de cobalt sous forme de dicobalt octacarbonyle. La température de réaction était de 215°C et la pression dans l'autoclave a été maintenue entre 145 et 155 bars par des recharges périodiques d'un mélange $CO/H_2$ : 1/2. En 30 minutes de réaction on a obtenu 4,15 g d'ethanol et 2,9 g d'oxyde de méthyle et d'éthyle. Pr : 130 g/h × 1 et 450 g/h × g (Co).

Exemple 4

En suivant le mode opératoire décrit précédemment on a chargé 100 ml de méthanol, 0,128 m At-g de cobalt sous forme d'iodure de cobalt, 1,66 m At-g de ruthénium sous forme d'iodure de ruthénium, 12 m Mol d'iodure de potassium. La température de réaction était de 205°C et la pression dans l'autoclave a été maintenue entre 180 et 250 bars par des recharges périodiques d'un

mélange $CO/H_2$ équimoléculaire. En 40 minutes de réaction on a obtenu 11,2 g d'éthanol et 7,7 g d'oxyde de méthyle et d'éthyle. La productivité en éthanol réalisable est de 270 g/h x 1 et de 3600 g/h x g (Co).

Exemple 5

En reproduisant le mode opératoire décrit précédemment on a chargé 3,58 m Mol d'iodure de méthyle, 90 ml de méthanol, 10 ml d'eau, 12 m Mol de bromure de sodium, 0,66 m At-g de ruthénium sous forme de triruthénium dodécacarbonyle, et 0,132 m At-g de cobalt sous la forme de dicobalt octacarbonyle.

La température de réaction était de 215°C et la pression dans l'autoclave a été maintenue entre 230 et 250 bars par des recharges périodiques d'un mélange $CO/H_2$ . 1/2. En 40 minutes de réaction on a obtenu 5,24 g d'éthanol et 1,9 g d'oxyde de méthyle et d'éthyle. La productivité en éthanol réalisable est de 100 g/h x 1 et de 1300 g/h x g (Co).

Exemple 6

En reproduisant le mode opératoire décrit précédemment on a chargé 12 m Mol d'iodure de lithium, 3,85 m Mol de bromure de butyle, 0,117 m At-g de cobalt sous forme de dicobalt octacarbonyle, 0,66 m At-g de ruthénium sous la forme de triruthénium dodécarbonyle, 90 ml de méthanol et 10 ml d'eau.

La température de réaction était de 225°C et la pression dans l'autoclave a été maintenue entre 200 et 235 bars au moyen de recharges périodiques d'un mélange $CO/H_2$ : 1/2. En 40 minutes de réaction on a obtenu 10,4 g d'éthanol et 0,5 g d'oxyde de méthyle et d'éthyle. La productivité en éthanol réalisable est de 170 g/h x 1 et de 2500 g/h x g (Co).

Exemple 7

En suivant le mode opératoire décrit précédemment on a chargé 95 ml de méthanol, 5 ml d'eau, 0,126 m At-g de cobalt sous la forme de dicobalt octacarbonyle, 0,66 m At-g de ruthénium sous forme de triruthénium dodécarbonyle 1,5 m Mol d'iodure de sodium et 3,46 m Mol d'iodure de méthyle.

La température de réaction était de 215°C et la pression dans l'autoclave a été maintenue entre 220 et 260 bars au moyen de recharges périodiques d'un mélange $CO/H_2$. En 30 minutes de réaction on a obtenu 11,7 g d'éthanol et 5,45 g d'oxyde de méthyle et d'éthyle. La productivité en éthanol réalisable est de 330. g/h x 1 et de 4400 g/h x g (Co).

Exemple 8

En suivant le mode opératoire décrit précédemment on a chargé 12 ml Mol d'iodure de tétraéthylammonium, 3,57 m Mol d'iodure de méthyle 0,126 m At-g de cobalt sous forme de dicobalt octacarbonyle, 0,66 m At-g de ruthénium sous la forme de triruthénium dodécacarbonyle, 90 ml de méthanol et 10 ml d'eau.

La température était de 220°C et la pression dans l'autoclave a été maintenue entre 210 et 260 bars au moyen de recharges périodiques d'un mélange $CO/H_2$ : 1/2. En 40 minutes de réaction on a obtenu 11,5 g d'éthanol et 3,40 g d'oxyde de méthyle et d'éthyle. La productivité en étanol réalisable est de 220g/h x 1 et de 3000 g/h x g (Co).

Exemple 9

En utilisant le mode opératoire décrit prédédemment on a chargé 1,3 m At-g de ruthénium sous forme de triruthénium dodécacarbonyle, 0,12 m At-g de cobalt sous la forme de dicobalt octacarbonyle, 90 ml de méthanol, 10 ml d'eau, 3,64 m Mol d'iodure de méthyle, 6 m Mol d'iodure de sodium. Après fermeture de l'autoclave on a établit une pression de 140 bars à l'aide d'un mélange $CO/H_2$ : 1/2. Après mise en route de l'agitation, on porte l'autoclave à 215°C. La pression dans l'autoclave atteint alors 220 bars, et elle est maintenue entre 220 et 260 bars par des recharges périodiques de ce mélange $CO/H_2$. Dans ces conditions et après 1 heure 15 minutes de réaction on a obtenue 14,1 g d'éthanol et 4,8 g d'oxyde de méthyle et d'éthyle. La productivité en éthanol réalisable est de 150 g/h x 1 et de 2100 g/h x 9 (Co).

Exemple 10

On a reproduit l'exemple 7, en chargeant une quantité double d'iodure de sodium. Toutes conditions égales par ailleurs on a obtenu 13,2 g d'éthanol et 5,10 g d'oxyde de méthyle et d'éthyle. La productivité en éthanol réalisable est de 350 g/h x 1 et de 4700 g/h x g (Co).

**Revendications**

1. Procédé d'hydrocarbonylation du méthanol en éthanol, en phase liquide, à une température d'au moins 180°C, sous une pression totale comprise entre 50 et 400 bars, en présence de cobalt et de ruthénium caractérisé en ce que la réaction est conduite en présence d'au moins un halogénure ionique dont le cation est choisi dans le groupe constitué par les cations des métaux alcalins, les cations des métaux alcalino-terreux, les cations ammonium quaternaire et les cations phophonium quaternaire, et d'au moins un halogénure d'alkyle, le rapport molaire $X^-/Co$, $X^-$ provenant de l'halogénure ionique, étant supérieur ou égal à 5, le rapport molair X/Co, X provenant de l'halogénure d'alkyle, étant supérieur ou égal à 2, et le rapport molaire Ru/Co étant supérieur à 2.

2. Procédé selon la revendication 1, caractérisée en ce que l'halogénure d'alkyle est choisi parmi les chlorures, les bromures et les iodures d'alkyles ayant de 1 à 4 atomes de carbone dans la molécule.

3. Procédé selon la revendication 2, caractérisé en ce que l'halogénure d'alkyle est un halogénure de méthyle.

4. Procédé selon la revendication 3, caractérisé en ce que l'halogénure de méthyle est, pour tout ou partie produit in situ à partir d'au moins un composé choisi dans le groupe constitué par le chlore, le brome et l'iode moléculaires, les acides halohydriques correspondants, le bromure et l'iodure de cobalt, le bromure et l'iodure de ruthénium.

5. Procédé selon l'une quelconque des revendications, précédentes, caractérisé en ce que le cation de l'halogénure ionique est choisi parmi les cations alcalins et les cations alcalino-terreux.

6. Procédé selon l'une quelconque des revendications précédentes, caractérisé en ce que l'halogénure ionique est un iodure.

7. Procédé selon l'une quelconque des revendications précédentes, caractérisé en ce que l'halogénure d'alkyle est un iodire.

8. Procédé selon l'une quelconque des revendications précédentes, caractérisé en ce que le rapport $X^-/Co$ est compris entre 10 et 100 environ.

9. Procédé selon l'une quelconque des revendications précédentes, caractérisé en ce que le rapport X/Co est compris entre 10 et 50.

10. Procédé selon l'une quelconque des revendications précédentes caractérisé en ce que la concentration du cobalt est comprise entre 0,1 et 100 et de préférence, entre 0,5 et 50 milliatomes-grammes par litre de milieu réactionnel.

11. Procédé selon l'une quelconque des revendications précédentes, caractérisé en ce que le rapport molaire Ru/Co est compris entre 5 et 20.

12. Procédé selon l'une quelconque des revendications précedentes, caractérisé en ce que la pression totale est comprise entre 100 et 350 bars.

13. Procédé selon l'une quelconque des revendications précédentes, caractérisé en ce que la temperature est comprise entre 200 et 240°C.

**Claims**

1. Process for the hydrocarbonylation of methanol to give ethanol, in the liquid phase, at a temperature of at least 180°C, under a total pressure of between 50 and 400 bars, in the presence of cobalt and ruthenium, characterised in that the reaction is carried out in the presence of at least one ionic halide, the cation of which is chosen from the group consisting of alkali metal cations, alkaline earth metal cations, quaternary ammonium cations and quaternary phosphonium cations, and in the presence of at least one alkyl halide, the molar ratio $X^-/Co$, $X^-$ originating from the ionic halide, being greater than or equal to 5, the molar ratio X/Co, X originating from the alkyl halide, being greater than or equal to 2 and the molar ratio Ru/Co being greater than 2.

2. Process according to claim 1, characterised in that the alkyl halide is chosen from amongst alkyl chlorides, bromides and iodides having from 1 to 4 carbon atoms in the molecule.

3. Process according to claim 2, characterised in that the alkyl halide is a methyl halide.

4. Process according to claim 3, characterised in that the methyl halide is totally or partially produced in situ from at least one compound chosen from the group consisting of molecular chlorine, molecular bromine and molecular iodine, the corresponding hydrohalic acids, cobalt bromide and iodide and ruthenium bromide and iodide.

5. Process according to any one of the preceding claims, characterised in that the cation of the ionic halide is chosen from amongst alkali metal cations and alkaline earth metal cations.

6. Process according to any one of the preceding claims, characterised in that the ionic halide is an iodide.

7. Process according to any one of the preceding claims, characterised in that the alkyl halide is an iodide.

8. Process according to any one of the preceding claims, characterised in that the ratio $X^-/Co$ is between about 10 and 100.

9. Process according to any one of the preceding claims, characterised in that the ratio X/Co is between 10 and 50.

10. Process according to any one of the preceding claims, characterised in that the concentration of the cobalt is between 0.1 and 100, and preferably between 0.5 and 50, milligram atoms per litre of reaction medium.

11. Process according to any one of the preceding claims, characterised in that the molar ratio Ru/Co is between 5 and 20.

12. Process according to any one of the preceding claims, characterised in that the total pressure is between 100 and 350 bars.

13. Process according to any one of the preceding claims, characterised in that the temperature is between 200 and 240°C.

**Patentansprüche**

1. Verfahren zum Hydrocarbonylieren von Methanol zu Äthanol in flüssiger Phase bei einer Temperatur von mindestens 180°C, unter einem Gesemtdruck von 50 bis 400 bar, in Gegenwart von Kobalt und Ruthenium, dadurch gekennzeichnet, daß das Verfahren in Gegenwart mindestens eines ionischen Halogenids, dessen Kation aus der Gruppe der Alkalikationen, Erdalkalikationen und quaternären Ammoniumsowie quaternären Phosphoniumionen ausgewählt ist, und in Gegenwart mindestens eines Alkylhalogenids bei einem Molverhältnis $X^-/Co$, wobei $X^-$ aus dem ionischen Halogenid stammt, von 5 oder darüber, einem

Molverhältnis X/Co, wobei X aus dem Alkylhalogenid stammt, von 2 oder darüber und einem Molverhältnis Ru/Co von über 2 ausgeführt wird.

2. Verfahren nach Anspruch 1, dadurch gekennzeichnet, daß das Alkylhalogenid aus der Gruppe der Alkylchloride, Alkylbromide und Alkyljodide mit 1 bis 4 Kohlenstoffatomen im Molekül ausgewählt wird.

3. Verfahren nach Anspruch 2, dadurch gekennzeichnet, daß das Alkylhalogenid ein Methylhalogenid ist.

4. Verfahren nach Anspruch 3, dadurch gekennzeichnet, daß das Methylhalogenid ganz oder teilweise in situ erzeugt wird, ausgehend von mindestens einer Verbindung aus der Gruppe bestehend aus molekularem Chlor, molekularem Brom und molekularem Jod, den entsprechenden Halogenwasserstoffsäuren sowie Kobaltbromid und Kobaltjodid und Rutheniumbromid und Rutheniumjodid.

5. Verfahren nach einem der vorangehenden Ansprüche, dadurch gekennzeichnet, daß das Kation des ionischen Halogenids unter den Alkalikationen und Erdalkalikationen ausgewählt wird.

6. Verfahren nach einem der vorangehenden Ansprüche, dadurch gekennzeichnet, daß das ionische Halogenid ein Jodid ist.

7. Verfahren nach einem der vorangehenden Ansprüche, dadurch gekennzeichnet, daß das Alkylhalogenid ein Alkyljodid ist.

8. Verfahren nach einem der vorangehenden Ansprüche, dadurch gekennzeichnet, daß das Verhältnis $X^-$/Co zwischen etwa 10 und 100 liegt.

9. Verfahren nach einem der vorangehenden Ansprüche, dadurch gekennzeichnet, daß das Verhältnis X/Co zwischen 10 und 50 liegt.

10. Verfahren nach einem der vorangehenden Ansprüche, dadurch gekennzeichnet, daß die Kobaltkonzentration 0,1 bis 100, vorzugsweise 0,5 bis 50 mgAtom je Liter Reaktionsmedium beträgt.

11. Verfahren nach einem der vorangehenden Ansprüche, dadurch gekennzeichnet, daß das Molverhältnis Ru/Co 5 bis 20 beträgt.

12. Verfahren nach einem der vorangehenden Ansprüche, dadurch gekennzeichnet, daß der Gesamtdruck 100 bis 350 bar beträgt.

13. Verfahren nach einem der vorangehenden Ansprüche, dadurch gekennzeichnet, daß die Temperatur 200 bis 240°C beträgt.